# EUROPÄISCHE PATENTSCHRIFT

(11) **EP 3 332 077 B1**
(45) Veröffentlichungstag und Bekanntmachung des Hinweises auf die Patenterteilung: **20.01.2021**
(21) Anmeldenummer: 16742231.0
(22) Anmeldetag: 18.07.2016
(51) Int. Cl.: E04H 3/08, A61G 10/00, A61G 3/00, E04H 5/02

(54) **MEDIZINISCHE CONTAINEREINHEIT ZUR PLANUNG UND FERTIGUNG EINES IMPLANTATS**
MEDICAL CONTAINER UNIT FOR PLANNING AND MANUFACTURING OF IMPLANTS
UNITÉ DE CONTENEUR MÉDICAL POUR LA PLANIFICATION ET LA FABRICATION D'IMPLANTS

(30) Priorität: 04.08.2015 DE 102015112774
(43) Veröffentlichungstag der Anmeldung: 13.06.2018
(73) Patentinhaber: Karl Leibinger Medizintechnik GmbH & Co. KG, 78570 Mühlheim (DE)
(72) Erfinder: LEIBINGER, Christian, 78570 Mühlheim (DE); MARTIN, Michael, 78570 Mühlheim (DE)
(74) Vertreter: Winter, Brandl, Fürniss, Hübner, Röss, Kaiser, Polte - Partnerschaft mbB
(86) Internationale Anmeldenummer: PCT/EP2016/067015
(87) Internationale Veröffentlichungsnummer: WO 2017/021128

(56) Entgegenhaltungen:
- WO-A1-00/68749
- WO-A1-2015/057722
- WO-A1-2015/184015
- DE-U1- 9 104 004
- FR-A1- 2 541 714
- US-A- 4 599 829
- US-A- 5 964 065
- US-A1- 2011 173 898
- US-A1- 2014 008 359
- US-A1- 2014 137 493

## Beschreibung

Die Erfindung betrifft eine medizinische Containereinheit (d.h. eine mobile Containereinrichtung) zur Planung und/oder Fertigung eines Implantats für einen Patienten, die mehrere Raumbereiche aufweist.

Aus dem Stand der Technik ist es bereits gattungsgemäß bekannt, prinzipiell Container für medizinische Zwecke zur Verfügung zu stellen. Beispielsweise offenbart die US 2011/0173898 A1 in diesem Zusammenhang eine selbstständige medizinische Versorgungseinheit.

Weiterer Stand der Technik ist aus der DE 20 2012 003 175 U1 bekannt, wobei ein modularer Raum in Gerüstbauweise, etwa ein Operationsraum im Medizinsektor, aufgebaut werden kann, wobei mehrere Tragprofile zur Ausbildung eines Deckengerüstes eingesetzt sind. Zudem ist Stand der Technik aus der FR 2 541 714 A1, der DE 91 04 004 U1, der US 2014/137493 A1, der WO 2015/057722 A1, der WO 2015/184014 A1, der US 5 964 065 A, der US 2014/008359 A1, der US 4 599 829 A und der WO 00/68749 A1 bekannt.

Bei dem aus dem Stand der Technik bekannten medizinischen Containern hat es sich jedoch als nachteilig herausgestellt, dass diese jeweils für sich genommen einen bestimmten Einsatzbereich verfolgen, wobei deren Raumbereiche feste, notwendige Bestandteile des jeweiligen Containers darstellen. Insbesondere sind diese Container jeweils nur im vollständig montierten Zustand des Containers in der Zusammenwirkung einsetzbar, um die gewünschte Funktion des Containers zu realisieren. Es ist daher bisher nicht möglich, auf die jeweiligen Platzbedingungen am Aufstellort des Containers zu reagieren und eine gesamtheitliche Containereinheit zur Verfügung zu stellen, die individuell an die Bedingungen dort anpassbar ist. Dadurch sind bisher für die jeweiligen Platzbedingungen am Aufstellort eigens angepasste Container zu produzieren.

Es ist daher die Aufgabe der vorliegenden Erfindung, diese aus dem Stand der Technik bekannten Nacheile zu beheben und insbesondere eine medizinische Containereinheit zur Verfügung zu stellen, die individuell auf die jeweils gewünschten Platzbedingungen am Einsatzbereich adaptierbar sein soll, wobei dessen Herstellung besonders kostengünstig gehalten werden soll.

Dies wird erfindungsgemäß durch die Merkmale des Anspruchs 1 gelöst. Dabei ist die Containereinheit u.a. mit mehreren, jeweils einen Raumbereich ausbildenden Containerteileinheiten ausgestattet, wobei jede Containerteileinheiten mit zumindest einem Mittel zur Planung und/oder Fertigung eines (vorzugsweise patientenspezifischen) Implantats ausgestattet und an zumindest zwei Seiten durch selbsttragende Unterteilkonstruktionen ausgestaltet / begrenzt ist. Dadurch sind die Raumbereiche jeweils durch individuell / eigenständig / selbstständig selbsttragende Container(raum)teileinheiten ausgestaltet / gebildet. Auch sind somit Raumbereiche geschaffen, die in Form der Containerteileinheiten unabhängig voneinander positionierbar / voneinander trennbar angeordnet sind.

Durch diese Anordnung ist es möglich, in Abhängigkeit der Grundmaße der Containerteileinheiten unterschiedlich hohe, breite sowie lange medizinische Containereinheiten aufzubauen. Dadurch ist ein Container / eine Containereinheit besonders vielseitig ausgestaltbar.

Für die Behandlung und Heilung des Patienten ist es vorteilhaft wenn patientenspezifisch , anatomisch angepasste Implantate möglichst schnell zur Verfügung stehen und damit der Zeitraum zwischen Patientenaufnahme und Operation minimiert werden kann. Darüber hinaus ist es vorteilhaft wenn zur Vorabüberprüfung der Operationstechnik dem Arzt schnell Modelle der Implantate zur Verfügung gestellt werden können. Durch die Integration aller Planungs- und/oder Fertigungsschritte in eine transportable Containereinheit entfallen Transportzeiten. Des Weiteren besteht die Möglichkeit der direkten unmittelbaren Kommunikation zwischen dem behandelndem Arzt und dem die Planungs- und/oder Fertigungseinheit betreffenden Techniker.

Weitere vorteilhafte Ausführungsformen sind in den Unteransprüchen beansprucht und nachfolgend näher erläutert.

Ist vorteilhafterweise jede Containerteileinheit mit zumindest einer weiteren Containerteileinheit wieder abnehmbar verbunden, ist der Container / die Containereinheit im aufgestellten Zustand besonders robust ausgebildet, gleichzeitig jedoch die individualisierte außengeometrische Anpassung rasch durchführbar.

Weiterhin ist es dabei auch zweckmäßig, wenn jede Containerteileinheit ein selbsttragendes Grundgerüst aufweist, an dem mehrere Unterteilkonstruktionen vorhanden sind, an denen mehrere Seitenwände, eine Decke und/oder einen Boden ausbildende Plattenabschnitte (vorzugsweise als Paneele ausgebildet) und/oder diese tragende Balken angebracht / befestigt sind. Dadurch ist gewährleitet, dass jede Containerteileinheit an sich / im einzelnen noch stabiler ist und ihr einzelner Raumbereich, unabhängig von den anderen Raumbereichen, aufstellbar / erzeugbar ist. Dies hat insbesondere Vorteile für den Transport des Containers / der Containereinheit durch das Zerlegen in die einzelnen Raumbereiche, die dann besonders platzsparend auf dem jeweiligen Transportfahrzeug platzierbar sind.

Ist das Grundgerüst in diesem Zusammenhang aus einem Metallwerkstoff, vorzugsweise einem Stahlwerkstoff hergestellt, kann das Grundgerüst besonders kompakt ausgebildet werden, wobei es trotzdem die notwendige Tragelast aufnehmen kann. Vorzugsweise ist das Grundgerüst durch mehrere miteinander verbundene Tragbalken aufgebaut oder durch weitere Tragbalken / Balken erweitert. Dadurch sind die Containerteileinheiten an sich ebenfalls besonders dünnwandig ausbildbar.

Sind die Plattenabschnitte in diesem Zusammenhang aus einem Leichtmetallwerkstoff, vorzugsweise einem Aluminiumwerkstoff, oder besonders bevorzugt aus einem Kunststoff hergestellt, ist die Verkleidung / Wandung des Containers / der Containereinheit auch besonders einfach und kostengünstig ausgebildet.

Weiter von Vorteil ist es, wenn einer der Raumbereiche / ein erster Raumbereich / eine der Containerteileinheiten / eine erste Containerteileinheit mit einer Hygieneeinheit umfassend ein Waschbecken, eine Dusche und/oder eine Toilettenschüssel, und/oder mit einer Büroeinheit, umfassend ein Büromobiliar, ausgestattet ist. Vorzugsweise ist dieser (erste) Raumbereich dabei sowohl mit der Hygieneeinheit als auch mit der Büroeinheit ausgebildet, wobei diese dann mittels einer (vorzugsweise wiederabnehmbaren) Innenwand voneinander räumlich abgegrenzt sind. Dadurch ist ein Raumbereich besonders effektiv genutzt.

In diesem Zusammenhang ist es auch weiter vorteilhaft, wenn einer der Raumbereiche / ein zweiter Raumbereich / eine der Containerteileinheiten / eine zweite Containerteileinheit mit einer (Implantat-)Planungseinheit umfassend einen eine Zeichensoftware (d.h. ein CAD-Programm) aufweisenden Rechner, und/oder mit einer Reinigungs- und/oder Verpackungseinheit für das Implantat ausgestattet ist. Vorzugsweise ist dieser (zweite) Raumbereich sowohl mit der Planungseinheit als auch mit der Reinigungs- und/oder Verpackungseinheit ausgebildet, wobei diese dann mittels einer (vorzugsweise wiederabnehmbaren) Innenwand voneinander räumlich abgegrenzt sind. Dadurch ist auch ein zweiter Raumbereich besonders effektiv genutzt.

Ist weiterhin einer der Raumbereiche / ein dritter Raumbereich / eine der Containerteileinheiten / eine dritte Containerteileinheit mit einer Handarbeitseinheit umfassend eine Werkbank, und/oder mit einer Oberflächenbearbeitungseinheit für das Implantat ausgestattet, ist auch ein solcher Raumbereich besonders nützlich ausgebildet. Die Handarbeitseinheit und die Oberflächenbearbeitungseinheit sind wiederum vorzugsweise in diesem (dritten) Raumbereich angeordnet und mittels einer weiteren (vorzugsweise wiederabnehmbaren) Innenwand voneinander getrennt.

Weiter zweckmäßig ist es, wenn einer der Raumbereiche / ein vierter Raumbereich / eine der Containerteileinheiten / eine vierte Containerteileinheit mit einer Fertigungseinheit zum maschinellen Fertigen des Implantates umfassend eine additive Fertigungsvorrichtung / -maschine, eine Sintervorrichtung / -maschine, eine Laser-Schmelz-Vorrichtung / -maschine und/oder eine Zerspanungsvorrichtung / -maschine, wie eine Fräsvorrichtung / -maschine, ausgestattet ist. Dadurch ist auch ein vierter Raumbereich besonders effektiv genutzt. Zudem wird dadurch der Nutzen des Containers weiter verbessert, da patientenspezifische Implantate schnellst möglichst dem Chirurgen vor Ort / im Krankenhaus zur Verfügung gestellt werden können.

Weiter ist es vorteilhaft, wenn die Containerteileinheiten einzeln oder weiter bevorzugt paarweise stapelbar / übereinander (wiederabnehmbar) stapelbar oder (wiederabnehmbar) aneinander anreihbar sind. Besonders bevorzugt sind die Containerteileinheiten jedoch derart ausgestaltet, dass sie sowohl nebeneinander / horizontal (wiederabnehmbar) verbindbar / anreihbar sind, als auch übereinander / vertikal stapelbar / miteinander (wiederabnehmbar) verbindbar sind. Dadurch können besonders vielfältige Container-Ausgestaltungen umgesetzt werden.

Im Weiteren ist es von Vorteil, wenn jede Containerteileinheit für die Aufnahme von zumindest einer Innenwand, vorzugsweise von mehreren wiederabnehmbaren Innenwänden ausgestaltet / vorbereitet ist.

Auch betrifft die Erfindung ein Verfahren zum Planen und/oder Fertigen eines Implantats, mit einzelnen (Planungs- und/oder Fertigungs-)Schritten, die bspw. ausschließlich innerhalb eines Krankenhauses und einer an diesem angegliederten Containereinheit nach einem der vorhergehend beschriebenen Ausführungen ablaufen.

In anderen Worten ausgedrückt, ist somit erfindungsgemäß eine mobile Implantats-Produktions-Containereinheit umgesetzt, die eine Produktionseinrichtung für die Herstellung von Implantaten bereitstellt und individuell an die gegebenen räumlichen Möglichkeiten am Einsatzort adaptierbar ist. Dieser Container / diese Containereinheit ist dann vorzugsweise bei seiner / ihrer Montage als Aufbau auf einem Fahrzeug und/oder stationär bei einem Aufstellen an einem bestimmten Ort, aufgebaut. Damit können patientenspezifische Implantate gefertigt und ohne lange Beschaffungs- und Versandwege schnellstmöglich bereitgestellt werden. Die Produktionseinrichtung /die Containereinheit kann an einer zentralen Stellen (etwa in Ballungsräumen) oder aber auch innerhalb eines Klinikums aufgestellt werden. Durch ein dem Chirurgen zur Verfügung gestelltes Software-Planungs-Tool werden die Implantate basierend auf CT-Daten (Patienten Scan) geplant und an die ortsnahe Produktionseinrichtung transferiert. Dort wird das Implantat dann maschinell gefertigt und an den Chirurgen übergeben.

Der medizinische Container kann folglich entweder als fester Container in Form eines Büro-Containers oder als Aufbau auf ein Service-Fahrzeug einer Kundendienstfirma montiert werden. Dadurch werden die patientenspezifischen Implantate schnellstmöglich dem Chirurgen zur Verfügung gestellt. Die Produktion von Implantaten wird mit qualifizierten und validierten Prozessen in Mobileinheiten / der Containereinheit sichergestellt, wobei eine Fertigung schnellstmöglich durchgeführt wird und insbesondere weltweit an allen Vertriebsstandorten ermöglicht werden kann. Zu den Vorteilen zählt daher, dass die Fertigung sowohl stationär als auch mobil ausgeführt werden kann, die Fertigung in modularer Bauweise zusammengestellt werden kann (pro Container / Containerteileinheit eine Technologie) und somit ideal auf den Bedarf skalierbar / erweiterbar ist. Es wird je nach benötigter Technologie die entsprechende Containereinheit mit den individuell notwendigen Raumbereichen / Containerteileinheiten zur Verfügung gestellt. Die Containereinheit ist dadurch besonders gut skalierbar.

Die einzelnen Abschnitte / Containerteileinheiten der Containereinheit sind relativ zu einander aber auch absolut gesehen skalierbar, um den Bedürfnissen zur Aufnahme von unterschiedlich großen Personengruppen, wie Medizinerin und Ingenieuren / Technikern, sowie unterschiedlich großen und häufigen Fertigungschargen Rechnung zu tragen.

Die Containereinheit ist zum Betrieb in unmittelbarer Nähe des Operationstraktes eines Krankenhauses vorgesehen, bemessen und beschaffen. Sie kann auch vorteilhafterweise direkt mit den Gebäudeteilen des Operationstraktes des Krankenhauses verbunden sein.

Die Erfindung wird nun nachfolgend anhand einer Figur näher erläutert, in welcher Figur eine schematische, vogelperspektivische Draufsicht auf eine erfindungsgemäße medizinische Containereinheit nach einem bevorzugten Ausführungsbeispiel dargestellt ist, in der die einzelnen Raumbereiche / Containerteileinheiten der Containereinheit sowie eine beispielhafte Anordnung derer erkennbar sind.

Die Figur ist lediglich schematischer Natur und dient ausschließlich dem Verständnis der Erfindung.

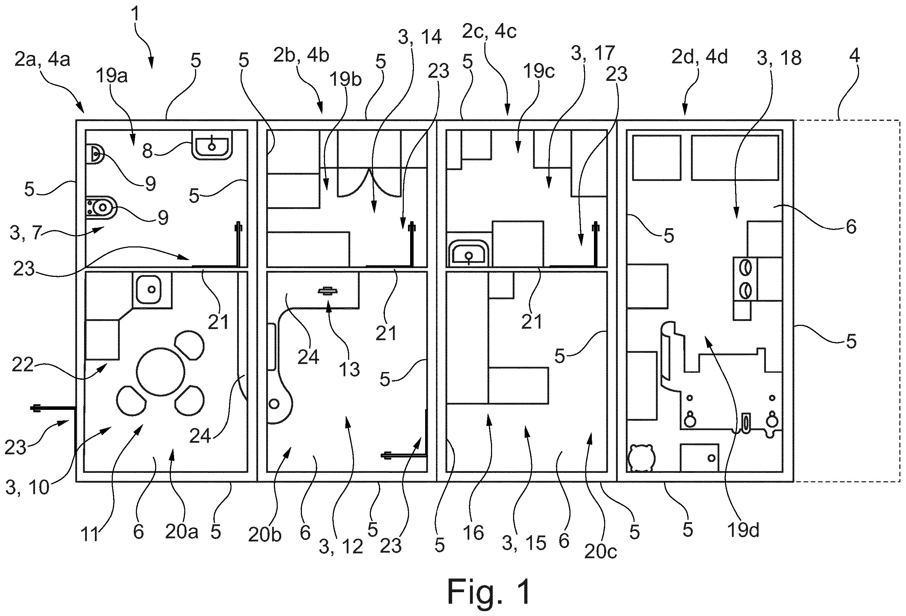

In Fig. 1 ist ein vorteilhaftes Ausführungsbeispiel einer erfindungsgemäßen medizinischen Containereinheit 1 anschaulich dargestellt. Die medizinische Containereinheit 1 dient in diesem Ausführungsbeispiel als eine mobile Planungs- sowie Fertigungsraumeinheit für ein Implantat eines Patienten. Diese mobile Containereinheit 1 ist als eigenständiger, einzelner Container ausgebildet und mittels eines Transportfahrzeuges transportierbar. Sie ist auf einer Aufstellfläche, bspw. einer Aufstellfläche innerhalb eines Krankenhauses aufstellbar. In einer weiteren Ausführung ist die Containereinheit 1 auch wieder abnehmbar als Fahrzeugaufbau mit einem Transportfahrzeug verbunden. Dabei bildet die Containereinheit 1 dann einen Aufbau eines Servicefahrzeuges aus.

Die Containereinheit 1 der erfindungsgemäßen Art, die auch als Container oder Containergesamteinheit bezeichnet ist, weist mehrere eigenständige, d.h. im Einzelnen selbsttragende Containerteileinheiten 4a bis 4d mit Unterteilkonstruktionen auf. Jede Containerteileinheit 4a bis 4d bildet einen Raumbereich 2a bis 2d. In dieser Ausführung sind mehr als zwei, nämlich vier Raumbereiche 2a bis 2d durch jeweils eine Containerteileinheit 4a bis 4d gebildet, wobei die Containerteileinheiten 4a bis 4d gemeinsam in ihrem verbundenen Zustand die Containereinheit 1 bilden. In weiteren Ausführungen sind jedoch auch mehr als vier (durch die gestrichelte Linie angedeutet), etwa 5 oder 6, oder weniger als vier, etwa zwei oder drei der Containerteileinheiten 4a bis 4d zu der Containereinheit 1 miteinander verbunden, ohne sich von dem grundsätzlichen Erfindungsgedanken zu entfernen.

In diesem Ausführungsbeispiel sind die vier eigenständigen Containerteileinheiten 4a bis 4d in Reihe nebeneinander angeordnet sowie aneinander wieder abnehmbar befestigt / miteinander wiederabnehmbar verbunden. Die Containerteileinheiten 4a bis 4d sind in Bezug auf ein Grundgerüst ihrer hier schematisch dargestellten vier Seitenwände 5 und einer hier der Übersichtlichkeit halber nicht weiter dargestellten Decke, sowie in Bezug auf ihren Boden 6 gleich / identisch aufgebaut. Jede der Containerteileinheiten 4a bis 4d bildet somit eine Art Modul aus, das durch das Befestigen mit zumindest einer weiteren Containerteileinheit 4a bis 4d zu der Containereinheit 1 verbunden ist. Jede Containerteileinheit 4 weist insbesondere zum Abstützen / Tragen der vier Seitenwände 5 sowie der Decke das Grundgerüst als Tragstruktur auf, das zumindest teilweise die Unterteilkonstruktion bildet. Dieses Grundgerüst ist mittels mehrerer Tragbalken in Form von Stahlträgern gebildet, die die Tragstruktur der einzelnen Containerteileinheiten 4a bis 4d bildet. Dadurch sind die vier Seitenwände 5 sowie die Decke durch einen Teil des integrierten Grundgerüstes aufgenommen / abgestützt und auch die Containerteileinheiten 4a bis 4d jeweils im Einzelnen selbsttragend ausgestaltet. Mit dem Grundgerüst sind dann wiederum mehrere plattenartige Abschnitte / Plattenabschnitte verbunden, um den Raumbereich 2a bis 2d zur Umgebung hin abzutrennen und die Ausbildung der vier Seitenwände 5, des Bodens 6 und der Decke zu gewährleisten.

Der Boden 6 des jeweiligen Raumbereiches 2a bis 2d / der jeweiligen Containerteileinheit 4a bis 4d ist aus einem ebenen Plattenabschnitt bereitgestellt / ausgeformt, der hier als eine einzige, die Grundmaße der jeweiligen der jeweiligen Containerteileinheit 4a bis 4d vorgebende Bodenplatte gebildet ist. Die Bodenplatte / der Boden 6 ist hier rechteckförmig ausgebildet. Die Bodenplatte besteht aus einem massiven Metallwerkstoff, vorzugsweise aus einem Stahlwerkstoff. Auch die Decke ist mittels eines solchen Plattenabschnittes oder alternativ mittels mehrerer Plattenabschnitte gebildet / ausgeformt, welche Plattenabschnitte dann mit dem Grundgerüst verbunden sind. Auch jede Seitenwand 5 ist auf übliche Weise mittels mehrerer Plattenabschnitte ausgeformt, die verkleidend an dem Grundgerüst angebracht sind. Insbesondere ist jede Containerteileinheit 4a bis 4d an einer zur Umgebung hin weisenden / einer der benachbarten Containerteileinheit 4a bis 4d abgewandten Seite vollständig mit Plattenabschnitten verkleidet, sodass ihr jeweiliger Raumbereich 2a bis 2d zur Umgebung hin abgegrenzt ist. Die Plattenabschnitte bilden somit gesamtheitlich im verbundenen Zustand der Containerteileinheiten 4a bis 4d zur Containereinheit 1 einen zur Umgebung hin abgetrennten Bereich / Raum aus. Das Grundgerüst sowie die Plattenabschnitte einer Containerteileinheit 4a bis 4d sind dabei derart angeordnet, dass sie in Bereichen zu einer benachbarten Containerteileinheit 4a bis 4d wieder abnehmbar / entfernbar sind. Dadurch ist es möglich, die einzelnen Raumbereiche 2a bis 2d räumlich miteinander zu verbinden.

Während das Grundgerüst aus einem Stahlwerkstoff / mehreren miteinander verbundenen Stahlträgern / Balken hergestellt ist bzw. mit Balken verbunden ist, sind die Plattenabschnitte vorzugsweise aus einem Aluminiumwerkstoff, alternativ auch aus einem Kunststoff hergestellt.

Auch der detaillierte Aufbau der einzelnen Raumbereiche 2a bis 2d ist in Fig. 1 zu erkennen. Dabei weist ein erster Raumbereich 2a / eine erste Containerteileinheit 4a eine Hygieneeinheit 7 (als ein erstes Mittel 3) innerhalb eines ersten Teilraumes 19a auf. Diese Hygieneeinheit 7 bildet eine Art Badezimmer aus und weist sowohl ein Waschbecken 8 als auch mehrere Toilettenschüsseln 9 auf. Über eine herausnehmbare / wiederabnehmbare Innenwand 21 ist ein zweiter Teilraum 20a des ersten Raumbereiches 2a wiederum räumlich getrennt von dem ersten Teilraum 19a angeordnet. In dieser Ausführung ist der zweite Teilraum 20a des ersten Raumbereiches 2a als ein Büro ausgebildet. Folglich weist der zweite Teilraum 20a eine Büroeinheit 10 (als ein zweites Mittel 3) auf, die ein Büromobiliar 11 sowie ein Küchenmobiliar 22 umfasst. In der Innenwand 21 zwischen dem ersten Teilraum 19a sowie dem zweiten Teilraum 20a ist eine Tür 23 angebracht, durch die Arbeitspersonal zwischen den Teilräumen 19a und 20a wechseln kann. Auch jene Seitenwand 5 des ersten Raumbereiches 2a / der ersten Containerteileinheit 4a, die auf einer einem zweiten Raumbereich 2b abgewandten Seite angeordnet ist und eine Außenwand ausbildet, weist eine als Außentür ausgebildete Tür 23 auf.

Eine zweite Containerteileinheit 4b ist mit ihrer Längsseite an der Längsseite der ersten Containerteileinheit 4a bündig angeordnet. Der durch die zweite Containerteileinheit 4b ausgebildete zweite Raumbereich 2b weist wiederum zwei Teilräume 19b und 20b auf, wobei der erste Teilraum 19b eine Reinigungs- und Verpackungseinheit 14 (als ein drittes Mittel 3) aufweist. Dabei ist zumindest eine Reinigungsvorrichtung zum Reinigen eines gefertigten Implantates vorhanden, bspw. ein Sprühreiniger oder ein Druckluftreiniger, und eine Verpackungsvorrichtung zum Verpacken des zuvor gereinigten Implantats, zum weiteren Transport an einen Patienten / an einen Chirurgen. Auch ist es denkbar nur entweder eine Reinigungs- oder eine Verpackungseinheit in diesem ersten Teilraum 19b anzubringen. Ein zweiter Teilraum 20b des zweiten Raumbereiches 2b weist wiederum eine Planungseinheit 12 (als ein viertes Mittel 3) auf. Diese Planungseinheit 12 weist wiederum einen elektronischen Rechner 13 auf, wovon hier der Übersichtlichkeit halber lediglich ein Monitor / bildliches Anzeigegerät von oben dargestellt ist. Mittels dieses Rechners 13, der auch über eine externe Datenverbindung, wie dem Internet, mit weiteren Rechnern / Datenbanken verbunden ist, werden zeitlich zuvor erfasste patientenspezifische 3D-Daten eines Patienten / eines zu behandelnden Körperbereiches des Patienten übermittelt / empfangen. Somit weist der Rechner 13 eine Empfangseinheit aus, mittels der 3D-Datensätze des jeweiligen Patienten bearbeitbar sind. Der Rechner 13 verfügt auch über eine Zeichen-Software, d.h. ein CAD-Programm, mittels dem ein Implantat anhand der übermittelten 3D-Geometriedaten des Patienten erstellt wird.

Die beiden Teilräume 19b und 20b des zweiten Raumbereiches 2b sind ebenfalls mittels einer herausnehmbaren / wiederabnehmbaren Innenwand 21 voneinander räumlich getrennt, in welcher Innenwand auch eine Tür 23 eingesetzt ist. In dieser Ausführung sind die Seitenwände 5 zwischen dem zweiten Teilraum 20a des ersten Raumbereiches 2a sowie dem zweiten Teilraum 20b des zweiten Raumbereiches 2b frei von Plattenabschnitten und somit räumlich miteinander verbunden, wie durch den sich von dem Teilraum 20b in den Teilraum 20a hinüber erstreckenden Tisch 24 der Planungseinheit 12 ersichtlich ist.

Eine dritte Containerteileinheit 4c ist wiederum mit einer Längsseite an einer der ersten Containerteileinheit 4a abgewandten Längsseite der zweiten Containerteileinheit 4b wieder abnehmbar angebracht. Auch der durch die dritte Containerteileinheit 4c gebildete dritte Raumbereich 2c ist zweigeteilt und weist einen ersten Teilraum 19c und einen zweiten Teilraum 20c auf. Der erste Teilraum 19c weist wiederum eine Oberflächenbearbeitungseinheit 17 (als ein fünftes Mittel 3) auf, mittels der eine Oberfläche des zuvor vorgefertigten Implantats bearbeitbar ist. Der erste Teilraum 19c des dritten Raumbereiches 2c ist mittels einer herausnehmbaren / wiederabnehmbaren Innenwand 21 sowie einer Tür 23 von dem zweiten Teilraum 20c abgegrenzt. In dem zweiten Teilraum 20c ist dann eine Handarbeitseinheit 15 (als ein sechstes Mittel 3) angeordnet, die u.a. eine Werkbank aufweist, um eventuelle Nacharbeiten an dem zuvor gefertigten Implantat durchzuführen. Der zweite Teilraum 20c des dritten Raumbereiches 2c ist über eine weitere Tür 23 mit dem zweiten Teilraum 20b des zweiten Raumbereiches 2b verbunden, wobei die aneinander anschließenden Seitenwände 5 der beiden Containerteileinheiten 4b und 4c im Bereich dieser Tür 23 ausgespart sind.

Auf einer der ersten und der zweiten Containerteileinheit 4a und 4b abgewandten Längsseite der dritten Containerteileinheit 4c ist wiederum eine vierte Containerteileinheit 4d mit ihrer Längsseite angeordnet und wieder abnehmbar an der dritten Containerteileinheit 4c angebracht. Ein durch diese vierte Containerteileinheit 4d ausgebildeter vierter Raumbereich 2d ist als ein einziger Raum 19d ausgebildet und weist mehrere eine Fertigungseinheit 18 (als ein siebtes Mittel 3) bildende Fertigungsvorrichtungen eines Implantates auf. Hierbei ist insbesondere das Anbringen bevorzugt einer additiven Fertigungsvorrichtung, etwa eines 3D-Druckers, weiter bevorzugt einer Sintervorrichtung, weiter bevorzugt einer Laser-Schmelzvorrichtung, besonders bevorzugt einer Zerspanungsvorrichtung, etwa einer Fräsvorrichtung in die Fertigungseinheit 18 umgesetzt. Mittels der Fertigungseinheit 18 bzw. der entsprechenden Fertigungsvorrichtung ist insbesondere ein medizinisches / chirurgisches Implantat herstellbar. Nach der Fertigung / Herstellung des Implantates innerhalb dieses vierten Raumbereiches 2d ist das Implantat der Handarbeitseinheit 15, der Oberflächenbearbeitungseinheit 17 und der Reinigungs- und Verpackungseinheit 14 zuführbar.

Neben diesen vier Raumbereichen 2a bis 2d sind beliebig viele weitere Raumbereiche durch weitere Containerteileinheiten 4, die wiederum mit den Containerteileinheit 4a bis 4d wieder abnehmbar verbindbar sind, zu der gesamtheitlichen Containereinheit 1 zusammensetzbar, was mit den gestrichelten Linien seitlich der vierten Containerteileinheit 4d in Fig. 1 schematisch dargestellt ist.

Jede Containerteileinheit 4a bis 4d weist auch eine gleiche Grundfläche auf, die durch den Boden 6 / die Bodenplatte vorgeben ist. Alle Raumbereiche 2a bis 2d weisen dabei eine rechteckige / rechteckförmige, ebene Grundfläche / Bodenfläche auf. Diese Bodenfläche ist besonders bevorzugt in ihrer Breite zwischen 40% bis 60 %, weiter bevorzugt halb so groß wie in ihrer Länge, etwa 3 m auf 6 m. In der Höhe sind die Containerteileinheiten 4a bis 4d jeweils vorzugsweise kleiner als 4 m hoch, so dass sie auch in einem üblicherweise ausgebildeten Raum eines Krankenhauses aufstellbar sind. Beispielhaft sind diese Containerteileinheiten 4a bis 4d / Raumbereiche 2a bis 2d jeweils zwischen 2,50 und 3,50, besonders bevorzugt um die 3 m / 3 m hoch. Bei einer Anordnung zweier paarweise nebeneinander angeordneter Containerteileinheit 4a bis 4d, etwa der ersten Containerteileinheit 4a und der zweiten Containerteileinheit 4b oder der dritten Containerteileinheit 4c und der vierten Containerteileinheit 4d ergibt sich dadurch in etwa eine quadratische Grundfläche des Zusammenbaus zweier Containerteileinheiten 4a bis 4d.

Dadurch ist ein Verfahren zum Planen und Fertigen des Implantats für einen Patienten / eine chirurgische Behandlung, mit einzelnen Planungs-und Fertigungsschritten, die bspw. ausschließlich innerhalb des Krankenhauses und der an diesem angegliederten Containereinheit 1 ablaufen, besonders effektiv umgesetzt. Die Fertigungsschritte werden ausschließlich im Container umgesetzt. Die Planung kann teilweise oder vollständig außerhalb des Containers erfolgen.

Alternativ zu der in der Reihe angeordneten Positionierung der Containerteileinheit 4a bis 4d zu der Containereinheit 1 gemäß Fig. 1 ist es auch möglich, diese teilweise, bspw. paarweise, oder allesamt aufeinander zu stapeln. Die einzelnen Raumbereiche 2a bis 2d sind daher derart ausgestaltet, dass sie nicht nur nebeneinander / horizontal miteinander verbindbar / anreihbar sind, sondern auch übereinander / vertikal stapelbar / miteinander verbindbar sind. Bei einer vertikalen Stapelung sind die aneinander angrenzenden Decken und Böden zweier Containerteileinheiten 4a bis 4d mit einer Treppe verbunden. Bei einer paarweise Stapelung zweier Containerteileinheiten 4a bis 4d, etwa der dritten und der vierten Containerteileinheit 4c und 4d, auf zwei anderen Containerteileinheiten 4a bis 4d, etwa der ersten und der zweiten Containerteileinheit 4a und 4b, bildet sich eine gesamtheitlich würfelfömige Containereinheit 1 aus. Die Containerteileinheiten 4a bis 4d sind dabei stets mit ihren benachbarten Containerteileinheiten 4a bis 4d (wiederabnehmbar) verbunden.

### Bezugszeichenliste

- 1: Containereinheit
- 2a: erster Raumbereich
- 2b: zweiter Raumbereich
- 2c: dritter Raumbereich
- 2d: vierter Raumbereich
- 3: Planungs- und/oder Fertigungsmittel
- 4a: erste Containerteileinheit
- 4b: zweite Containerteileinheit
- 4c: dritte Containerteileinheit
- 4d: vierter Containerteileinheit
- 5: Seitenwand
- 6: Boden
- 7: Hygieneeinheit
- 8: Waschbecken
- 9: Toilettenschüssel
- 10: Büroeinheit
- 11: Büromobiliar
- 12: Planungseinheit
- 13: Rechner
- 14: Reinigungs- und Verpackungseinheit
- 15: Handarbeitseinheit
- 16: Werkbank
- 17: Oberflächenbearbeitungseinheit
- 18: Fertigungseinheit
- 19a: erster Teilraum des ersten Raumbereiches
- 19b: erster Teilraum des zweiten Raumbereiches
- 19c: erster Teilraum des dritten Raumbereiches
- 19d: Raum des vierten Raumbereiches
- 20a: zweiter Teilraum des ersten Raumbereiches
- 20b: zweiter Teilraum des zweiten Raumbereiches
- 20c: zweiter Teilraum des dritten Raumbereiches
- 21: Innenwand
- 22: Küchenmobiliar
- 23: Tür
- 24: Tisch

## Patentansprüche

1. Medizinische Containereinheit (1) zur Planung und Fertigung eines patientenspezifischen Implantats, mit mehreren, jeweils einen Raumbereich (2a, 2b, 2c, 2d) ausbildenden Containerteileinheiten (4a, 4b, 4c, 4d), **dadurch gekennzeichnet, dass** ein Raumbereich (2b)
einer Containerteileinheit (4b) sowohl mit zumindest einem Mittel (3) zur Planung des Implantats in Form einer Planungseinheit (12) umfassend einen eine Zeichensoftware aufweisenden Rechner (13), als auch mit einer Reinigungs- und/oder Verpackungseinheit (14) ausgestattet ist und ein weiterer Raumbereich (2d) einer weiteren Containerteileinheit (4d) mit zumindest einem Mittel zur maschinellen Fertigung eines Implantats in Form einer Fertigungseinheit (18) ausgestattet ist, und wobei jede Containerteileinheit (4b, 4d) an zumindest zwei Seiten durch selbsttragende Unterteilkonstruktionen ausgestaltet ist.

2. Containereinheit (1) nach Anspruch 1, **dadurch gekennzeichnet, dass** jede Containerteileinheit (4a, 4b, 4c, 4d) mit zumindest einer weiteren Containerteileinheit (4a, 4b, 4c, 4d) wiederabnehmbar verbunden ist.

3. Containereinheit (1) nach Anspruch 1 oder 2, **dadurch gekennzeichnet, dass** jede Containerteileinheit (4a, 4b, 4c, 4d) ein selbsttragendes Grundgerüst aufweist, an dem mehrere Unterteilkonstruktionen vorhanden sind, an denen mehrere Seitenwände (5), eine Decke und/oder einen Boden (6) ausbildende Plattenabschnitte und/oder diese tragende Balken angebracht sind.

4. Containereinheit (1) nach Anspruch 3, **dadurch gekennzeichnet, dass** das Grundgerüst aus einem Metallwerkstoff hergestellt ist.

5. Containereinheit (1) nach Anspruch 3 oder 4, **dadurch gekennzeichnet, dass** die Plattenabschnitte aus einem Leichtmetallwerkstoff oder einem Kunststoff hergestellt sind.

6. Containereinheit (1) nach einem der Ansprüche 1 bis 5, **dadurch gekennzeichnet, dass** ein erster Raumbereich (2a) mit einer Hygieneeinheit (7) umfassend ein Waschbecken (8), eine Dusche und/oder eine Toilettenschüssel (9), und/oder mit einer Büroeinheit (10), umfassend ein Büromobiliar (11), ausgestattet ist.

7. Containereinheit (1) nach einem der Ansprüche 1 bis 6, **dadurch gekennzeichnet, dass** ein zweiter Raumbereich (2b) mit der Planungseinheit (12) umfassend den die Zeichensoftware aufweisenden Rechner (13) und die Reinigungs- und/oder Verpackungseinheit (14) zusätzlich mit einer Sterilisationseinheit ausgestattet ist.

8. Containereinheit (1) nach einem der Ansprüche 1 bis 7, **dadurch gekennzeichnet, dass** ein dritter Raumbereich (2c) mit einer Handarbeitseinheit (15) umfassend eine Werkbank (16), und/oder mit einer Oberflächenbearbeitungseinheit (17) ausgestattet ist.

9. Containereinheit (1) nach einem der Ansprüche 1 bis 8, **dadurch gekennzeichnet, dass** ein vierter Raumbereich (2d) mit der Fertigungseinheit (18) zum maschinellen Fertigen des Implantates umfassend eine additive Fertigungsvorrichtung, eine Sintervorrichtung, eine Laser-Schmelz-Vorrichtung und/oder eine Zerspanungsvorrichtung, wie eine Fräsvorrichtung, ausgestattet ist.

10. Containereinheit (1) nach einem der Ansprüche 1 bis 9, **dadurch gekennzeichnet, dass** die Containerteileinheiten (4a, 4b, 4c, 4d) übereinander stapelbar und/oder aneinander anreihbar sind.

## Claims

1. A medical container unit (1) for designing and manufacturing a patient-specific implant, comprising a plurality of container subunits (4a, 4b, 4c, 4d) each of which forms a sector (2a, 2b, 2c, 2d), **characterized in that**
a sector (2b) of a container subunit (4b) is equipped with a means (3) for designing the implant in the form of a design unit (12) comprising a computer (13) including drawing software as well as with a cleaning and/or packaging unit (14), and further sector (2d) of a further container subunit (4d) is equipped with at least one means for machine manufacturing an implant in the form of a manufacturing unit (18), and wherein each container subunit (4b, 4d) is formed by self-supporting partial substructures on at least two sides.

2. The container unit (1) according to claim 1, **characterized in that** each container subunit (4a, 4b, 4c, 4d) is re-detachably connected to at least one further container subunit (4a, 4b, 4c, 4d).

3. The container unit (1) according to claim 1 or 2, **characterized in that** each container subunit (4a, 4b, 4c, 4d) includes a self-supporting skeletal structure at which a plurality of partial substructures is present at which plate sections forming a plurality of sidewalls (5), a ceiling and/or a bottom (6) and/or beams supporting the same are arranged.

4. The container unit (1) according to claim 3, **characterized in that** the skeletal structure is made from a metallic material.

5. The container unit (1) according to claim 3 or 4, **characterized in that** the plate sections are made from a light-metal material or a plastic material.

6. The container unit (1) according to one of the claims 1 to 5, **characterized in that** a first sector (2a) is equipped with a sanitary unit (7) comprising a lavatory (8), a shower and/or a toilet bowl (9) and/or with an office unit (10) comprising office furniture (11).

7. The container unit (1) according to one of the claims 1 to 6, **characterized in that** a second sector (2b) is equipped with the design unit (12) comprising the computer (13) including the drawing software and the cleaning and/or packaging unit (14) is additionally equipped with a sterilization unit.

8. The container unit (1) according to one of the claims 1 to 7, **characterized in that** a third sector (2c) is equipped with a handcraft unit (15) comprising a workbench (16) and/or with a surface finishing unit (17).

9. The container unit (1) according to one of the claims 1 to 8, **characterized in that** a fourth sector (2d) is equipped with the manufacturing unit (18) for machine-manufacturing the implant comprising an additive manufacturing device, a sintering device, a laser fusing device and/or a cutting device such as a milling device.

10. The container unit (1) according to one of the claims 1 to 9, **characterized in that** the container subunits (4a, 4b, 4c, 4d) can be stacked on top of each other and/or can be stringed to each other.

## Revendications

1. Module médical formant conteneur (1) pour la planification et la fabrication d'un implant spécifique à un patient, avec plusieurs sous-modules de conteneur (4a, 4b, 4c, 4d) formant respectivement une région spatiale (2a, 2b, 2c, 2d), **caractérisé en ce qu'**une région spatiale (2b) d'un sous-module de conteneur (4b) est équipée d'au moins un moyen (3) de planification de l'implant sous la forme d'un module de planification (12) comprenant un ordinateur (13) avec logiciel de dessin ainsi que d'un module de nettoyage et/ou d'emballage (14) et une autre région spatiale (2d) d'un autre sous-module de conteneur (4d) est équipée d'au moins un moyen de fabrication mécanique d'un implant sous la forme d'un module de fabrication (18), et dans lequel chaque sous-module de conteneur (4b, 4d) est conçu sous forme de structures de compartimentage autoporteuses sur au moins deux côtés

2. Module formant conteneur (1) selon la revendication 1, **caractérisé en ce que** chaque sous-module de conteneur (4a, 4b, 4c, 4d) est relié de manière amovible à au moins un autre sous-module de conteneur (4a, 4b, 4c, 4d).

3. Module formant conteneur (1) selon la revendication 1 ou 2, **caractérisé en ce que** chaque sous-module de conteneur (4a, 4b, 4c, 4d) présente une ossature autoporteuse au niveau de laquelle se trouvent plusieurs structures de compartimentage au niveau desquelles sont fixés plusieurs parois latérales (5), un plafond et/ou des sections de plaque formant un plancher (6) et/ou des poutres supportant lesdites sections de plaque.

4. Module formant conteneur (1) selon la revendication 3, **caractérisé en ce que** l'ossature est réalisée en un matériau métallique.

5. Module formant conteneur (1) selon la revendication 3 ou 4, **caractérisé en ce que** les sections de plaque sont réalisées en un matériau métallique léger ou en une matière plastique.

6. Module formant conteneur (1) selon l'une quelconque des revendications 1 à 5, **caractérisé en ce qu'**une première région spatiale (2a) est équipée d'un module d'hygiène (7) comprenant un lavabo (8), une douche et/ou une cuvette de toilettes (9), et/ou d'un module de bureau (10) comprenant du mobilier de bureau (11).

7. Module formant conteneur (1) selon l'une quelconque des revendications 1 à 6, **caractérisé en ce qu'**une deuxième région spatiale (2b) est équipée du module de planification (12) comprenant l'ordinateur (13) avec logiciel de dessin et le module de nettoyage et/ou d'emballage (14) est également équipé d'un module de stérilisation.

8. Module formant conteneur (1) selon l'une quelconque des revendications 1 à 7, **caractérisé en ce qu'**une troisième région spatiale (2c) est équipée d'un module de travail manuel (15) comprenant un établi (16) et/ou d'un module de traitement de surface (17).

9. Module formant conteneur (1) selon l'une quelconque des revendications 1 à 8, **caractérisé en ce qu'**une quatrième région spatiale (2d) est équipée du module de fabrication (18) permettant la fabrication mécanique de l'implant et comprenant un dispositif de fabrication additive, un dispositif de frittage, un dispositif de fusion laser et/ou un dispositif d'usinage par enlèvement de copeaux tel qu'un dispositif de fraisage.

10. Module formant conteneur (1) selon l'une quelconque des revendications 1 à 9, **caractérisé en ce que** les sous-modules de conteneur (4a, 4b, 4c, 4d) peuvent être empilés les uns sur les autres et/ou alignés les uns à côté des autres.
